# EUROPEAN PATENT SPECIFICATION

(11) **EP 2 468 201 B1**
(45) Date of publication and mention of the grant of the patent: **08.10.2014**
(21) Application number: 12158902.2
(22) Date of filing: 12.08.2008
(51) Int. Cl.: B26D 3/00, B26D 7/01, B26F 1/14, B26F 1/02, A61B 17/70, B26F 1/12, B26F 1/00

(54) **Flexible stabilization device including a rod and tool for manufacturing the rod**
Flexible Stabilisierungsvorrichtung mit einer Stange und einem Werkzeug zur Herstellung der Stange
Dispositif de stabilisation flexible incluant une tige et outil de fabrication de la tige

(43) Date of publication of application: 27.06.2012
(62) Divisional of application: 08014378.7
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Pabst, Martin, 78166 Donaueschingen (DE); Dannecker, Berthold, 78112 St. Georgen (DE)
(74) Representative: Prüfer & Partner GbR European Patent Attorneys

(56) References cited:
- US-A- 4 281 546
- US-A- 4 784 638
- US-A1- 2004 236 327
- US-A1- 2005 261 686
- US-A1- 2007 093 814
- US-A1- 2007 225 710
- US-A1- 2007 299 442
- US-A1- 2008 177 320

## Description

The invention relates to a flexible stabilization device for connecting at least two bone anchoring devices which are attached to vertebrae of the spinal column.

Such flexible stabilization devices may generally comprise a rod which is provided with a considerable degree of stiffness in order to stabilize the spinal column. The bone anchoring devices may include receiving parts, which are provided with recesses to receive the rod, and fixation screws to accomplish a tight connection between the receiving parts and the rod. The receiving parts are further connected with, or integrally formed with bone screws, which may be screwed in to the adjacent vertebrae, e.g., pedicles. Thereby, multiple anchoring devices may be connected using the rod as described above.

In recent years there had been many efforts to provide a connection rod with flexible behaviour. The flexibility allows the spinal column to be moved in a controlled manner.

As an example, WO 1996/016608 A1 shows a vertebral instrumentation rod which is made of a basically rigid material, i.e., a metal or metal alloy. Hence, the rod is rigid in a first part where a cross section is generally cylindrical. However, in a second part, the rod is substantially flat to allow for flexion in a sagittal plane while impeding flexion in the frontal plane. In a transition zone, the degree flatness smoothly increases from the first towards the second part.

US 2005/085815 A1, by Applicant, shows a rod-shaped element wherein an flexible section is integrally formed with two adjacent rigid sections. The rod-shaped element may be composed of a metal material, and the flexible section may be provided by a coil spring allowing for flexion. In one specific embodiment, a cross section of the flexible section, i.e., of the coil spring, is flattened in one direction in achieve desired flexion properties.

US 2007/049937 A1, by Applicant, shows in one embodiment a rod-shaped implant element wherein an flexible section is connected between stiff portions. The flexible section is made from, e.g., polyurethane or polysiloxane, whereas the stiff portions are made from, e.g., titanium. The connection is provided by threads. Further, the flexible section may have a reduced or increased thickness depending on a desired amount of compression or extension capabilities.

US 2007/270821 A1 shows a vertebral stabilizer, which includes a connector formed as a single piece construction. The connector has an annular section of a reduced diameter, as compared with interposed sections, in order to enable stretching of the same. The connector may be formed from flexible fiber material.

US 2003/191470 A1 shows a dynamic fixation system wherein a rod may be shaped and thinned to function as a spring or pivot. The rod may be connected with pedicle screws via connectors while elastomer materials may be used for the rod, although not being preferred. A stepped flattened cross sectional profile may be obtained depending on the desired flexion or torsion characteristics.

US 2004/0236327 A1 discloses a flexible element of a spinal stabilization system that has a flexible and cylindrical middle portion formed with a spiral pattern of spaces or gaps. The ends of the element are flattened and eyelets are punched through the flattened ends.

US 2008/0177320 A1 discloses a vertebral rod made from polymer materials such as PEEK or UHMWPE. The rod is formed with one or more notches which extend into the rod from opposing multiple sides. A staggered or overlapping arrangement is possible. The notches are filled with a fill material, for example carbon fiber, polycarbonates, silicone or PEEK, or combinations thereof, including a Young's Modulus that is less than that of the rod. The notches are shaped sized and positioned to facilitate vertebral movement in first and second panes and prevent or inhibit movement in a third plane.

It is an object of the invention to further improve known techniques of stabilizing human vertebrae and to provide a flexible stabilization device that covers desired flexion and torsion characteristics while reducing the efforts and costs of manufacturing the same.

These and other objects are solved by a flexible stabilization device including a rod according to appended claim 1. Further aspects and embodiments become apparent from the appended claims.

According to one aspect of the invention, a flexible stabilization device comprises a flexible rod made of an elastomer material, and a surface of the rod in which is provided at least one first recess that is formed by means of separating material from the rod. The recess is provided as a groove comprising a flat portion at a bottom surface thereof.

The recess may influence the local bending and/or torsional properties of the rod. In one aspect, the separation may yield a directed removal of material from the rod along a first direction. Hence, the recess formed accordingly may affect the flexural rigidity of the rod in one particular (second) direction, while the original flexural rigidity of the rod may be maintained in another direction, e.g., perpendicular to the first direction.

Therefore, the recess applied to the rod may serve to achieve a reduced flexural rigidity of the rod along an orientation direction according to the specific needs of the concerned spinal column. For example, the overall flexural rigidity of the rod may be reduced at a specific position along the spinal column in order to allow for an improved flexion in, e.g., the frontal or the sagittal plane.

The separation process refers to punching out material from the rod using a punching or cutting tool. This further allows implementing multiple punched recesses to be formed along the rod surface according to the desired needs - under consideration of local flexural strength and its orientation with respect to the rod, or spinal column. According to specific embodiments of this aspect, the depth, length, mutual orientation or the number density of the multiple recesses can be varied along the rod to treat different parts of the spinal column in the most appropriate manner regarding flexion and/or torsion.

A tool which is not subject of the claimed invention includes a socket die with a first bore for receiving the rod connector, and at least one second bore for receiving a first punching press, and further includes the punching press, which fits within the at least one second bore being moveable with respect thereto. Thereby, the first and the at least one second bore intersect each other to enable punching out material from the flexible rod to form the recess due to the movable punching press.

One particular advantage of using a rod having punched recesses and employing a corresponding punching tool as described above may be found in that the shaping of the rod can be accomplished by the surgeon or another person prior to the actual surgery, since no complex injection molding method is required. The tool can also be used easily in the operating room environment.

Further features and advantages of the bone anchoring device will become apparent and will be best understood by reference of the following detailed description taken in conjunction with the accompanying drawings. Therein:
- Fig. 1: shows a schematic drawing of the spinal column, to which is added a stabilization device including a set of bone screws and one of three types of rods;
- Fig. 2: shows a rod in including recesses according to an embodiment in a perspective view;
- Fig. 3: same as Fig. 2, but in a top view, the rod being rotated by 90 degrees between the states shown;
- Fig. 4: a perspective view of a tool for separating material from an elastomer rod according to a first embodiment in a state before separation;
- Fig. 5: same as in Fig. 4, but in a state when the tool is operated to separate the material;
- Fig. 6: same as in Fig. 4, but in a cross-sectional representation;
- Fig. 7: same as in Fig. 5, but in a cross-sectional representation;
- Fig. 8: in a perspective view a second embodiment of a tool for separating material from an elastomer rod, wherein the state shown refers a first step of a separation method;
- Fig. 9: same as Fig. 8, but with reference to a second step;
- Fig. 10: same as Fig. 8, but with reference to a third step;
- Fig. 11: same as Fig. 8, but with reference to a fourth step;
- Fig. 12: shows further embodiments of recesses which may be implemented with a flexible rod by means of a separation method.

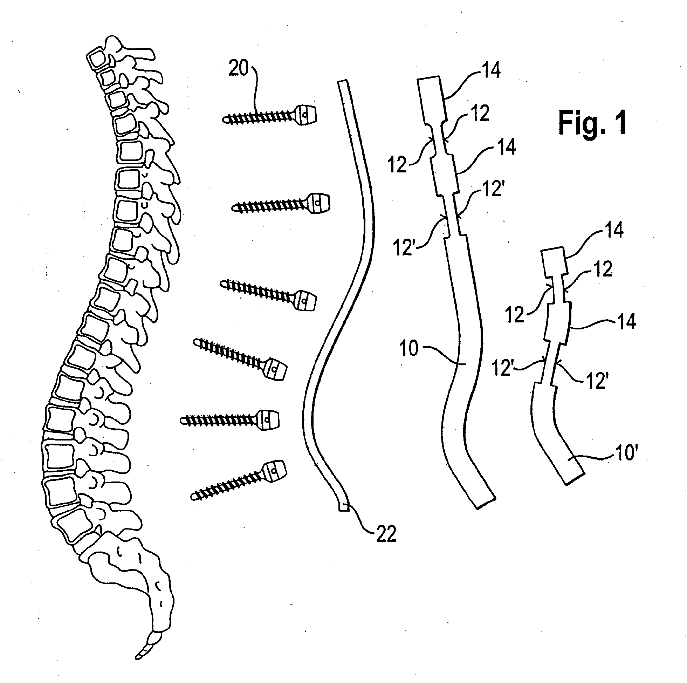

Fig. 1 shows in a schematic drawing an example of the spinal column, to which a dynamic stabilization device may be attached, that reflects one specific embodiment of the invention.

In a dynamic stabilization device, a flexible rod 22 may be employed to provide a fusion to vertebrae of the spinal column when the rod is clamped by respective bone anchoring devices 20. Thereby, the bone anchoring devices 20 are screwed in to specific vertebrae at appropriate height positions selected by the surgeon. The bone anchoring devices may be one of the monoaxial (i.e., the bone thread part and receiving part are rigidly fixed to each other) or polyaxial type (i.e., the bone thread part being pivotable with respect to an axis of receiving part prior to locking), but the present embodiments shall not be limited to the specific functions of the bone anchoring device.

The rod 22 which is schematically indicated in the center portion of Fig. 1 includes a constant diameter or thickness throughout its length and is made of an elastomer material. The thickness is chosen such as to provide a reasonable degree of stiffness or rigidity over its entire length. When this rod is clamped by corresponding receiving parts of the bone anchoring devices, a substantially rigid fusion between the vertebrae involved is achieved.

However, in various instances, it may be desirable to increase the flexibility of the rod. In one instance, the load which acts on vertebrae in a direct neighbourhood of fusioned parts of the spine may be too high. In order to relieve the load, the end part of the fusion can be provided with an enhanced degree of flexibility to enable a slight bending movement of the respective vertebrae.

Hence, in the present embodiment, a rod 10 made of an elastomer material is manufactured from the rod 22, wherein the rod 10 now includes recesses 12 formed on opposite sides of the rod. The recesses are formed by removal of material from the rod 22. Due to this removal the rod is thinned, as a result of which the bending flexibility increases locally. The outermost vertebrae are thus allowed to perform a slight movement depending on the load.

The opposing recesses 12 are formed between two respective clamping sections 14 of the rod 10, which are defined such as to be received by the receiving parts of respective bone anchoring devices 20 and thus include an appropriate length section of the rod 10.

In order to allow further portions of the spine to undergo a slight bending movement, an additional pair of recesses 12' is formed in the rod surface. The opposing recesses 12' may leave a same thickness of the rod as the recesses 12, while the length may be slightly extended, as an example.

In Fig. 1 there is also shown a rod 10' as an alternative embodiment which has the same features as the rod 10, but is provided with a comparatively shorter length.

The rod according to the embodiments includes a preferably bio-compatibe elastomer material. Examples of such materials which may be embodied herein are polyurethane, polysiloxane, Poly(styrene-block-isobutylene-block-styrene) (SIBS), or polycarbonate urethane (PCU).

It may be noted that the term "rod" as used herein basically denotes a rod-shaped element, which may be provided as a single piece rod as well as a multi-part composite element, that is put together to yield a fusion. In the latter case, those parts may for example be provided with corresponding threads to connect the corresponding pieces. Further, one of the parts may include the elastomer material while another part of the rod may include, e.g., a metal.

A case in which for example a single piece rod is formed from injection molding of two or more different elastomer material components shall also be encompassed by the invention, wherein the recesses are applied afterwards.

It may be noted that whether a recess is formed by later removal of material, in particular punching, or not may be clearly recognizable from the product: due to a slight deformation, or flow of stressed elastomer material during punching and/or heating for example, plane surfaces or straight lines which are formed thereby also may become slightly concave or curved, respectively. Further, score marks or miniature rims may form across the cut surface along the punching direction.

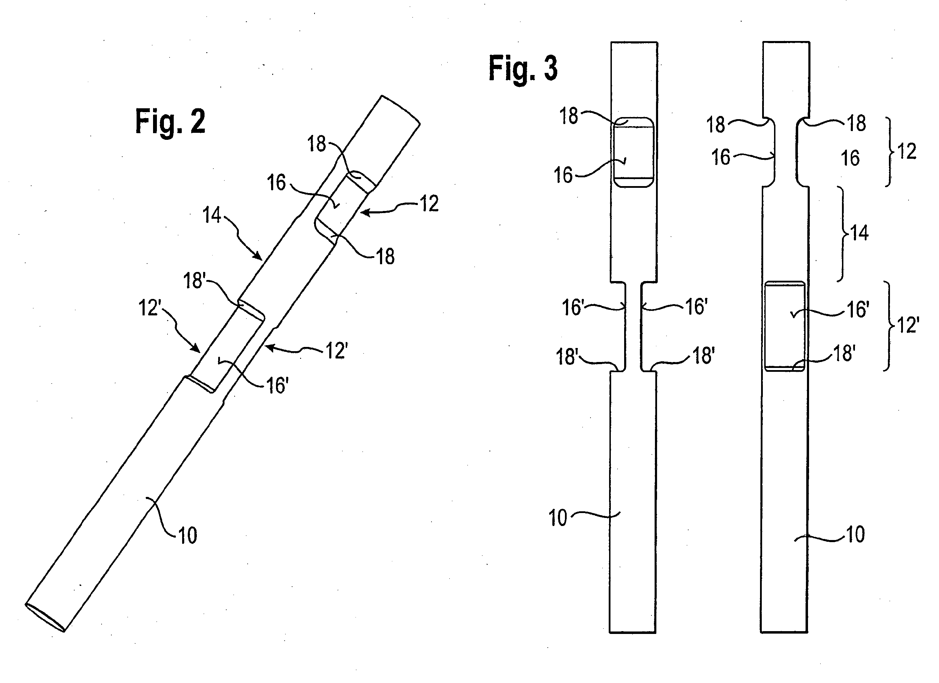

Figs. 2 and 3 show another embodiment of a rod 10 which also includes an elastomer material similar to that shown in Fig. 1. However, unlike in the previous embodiment the recesses 12 and 12' (or pair of recesses) are oriented in different directions with respect to each other by about 90 degrees.

The recesses 12, 12' have a well-defined shape of limited length. As becomes evident from Fig. 3, the recesses 12 include a flat portion 16 and two rounded end portion 18. The further recesses 12' also have a flat portion 16', while the end portions 18' are steeper and rise up sharply. In this embodiment, the flat portions 16' have a larger depth with respect to the surface than flat portions 16.

It may be noted that a rod 10 as described herein may also be formed with multiple recesses 12 or 12', all of which have the same shape - possibly with orientations with respect to the longitudinal axis of the rod, which differ from each other.

Despite the above mentioned presence of concave surfaces, score marks or miniature rims, it has been found that a punching operation may still fulfil the requirements with respect to surface roughness and punching accuracy quite satisfactorily, if a tool for punching a rod according to another aspect of the invention is used.

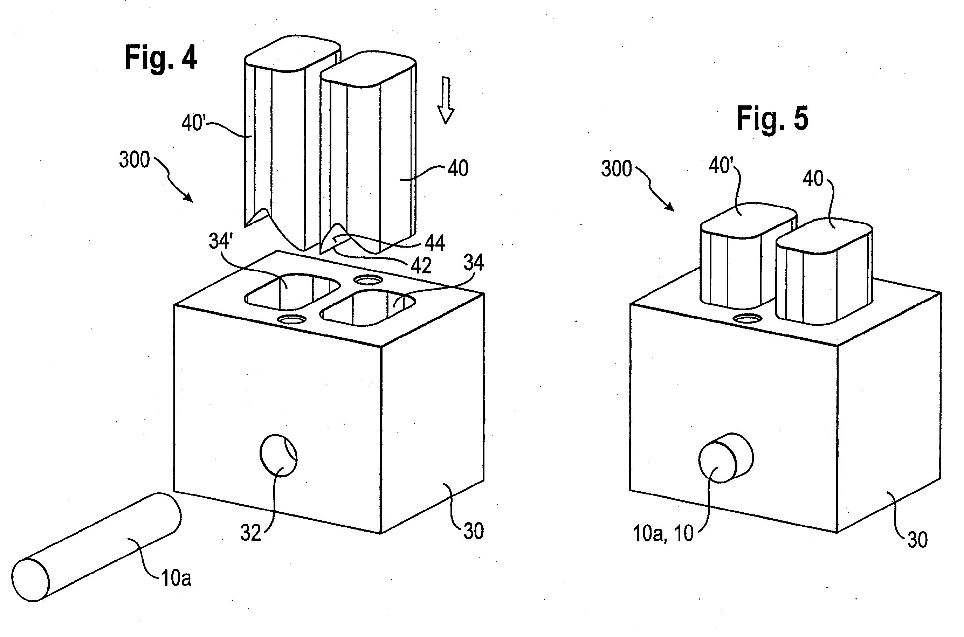

Figs. 4-7 show a first embodiment of a tool 300 for removing elastomer material from a rod 10a to manufacture one of the rods 10 shown in Figs. 1-3. The tool shown represents a punching tool. The tool comprises a socket die 30, which is provided with a bore 32 for insertion of the yet un-punched rod 10a, and with bores 34, 34' which receive each one of two punching presses 40, 40'. In this embodiment, the bore 32 extends through the socket die 30 in a horizontal direction while bore 34, 34' are oriented in a vertical direction. The rod 10a can be freely adjusted - i.e., pushed or rotated - in its position within bore 32 wherein the outer diameter of the rod 10a substantially corresponds to the inner diameter of the bore 32.

The punching presses 40, 40' are also shaped and sized to fit into the bores 34, 34'. As the bores 34, 34' are formed in parallel to each other, the orientation and guidance of the punching presses 40, 40' are also parallel. The bores 34, 34' intersect with the bore 32 such that the punching presses 40, 40' may cut material from rod 10a inserted in the bore 32. For this purpose, both presses are provided with cutting edges 42, 42' and cutting faces 44, 44' whose rake angle relative to the cutting direction serves to separate the removed elastomer material from the rod. The removed material may be discharged into a container not shown in the figures.

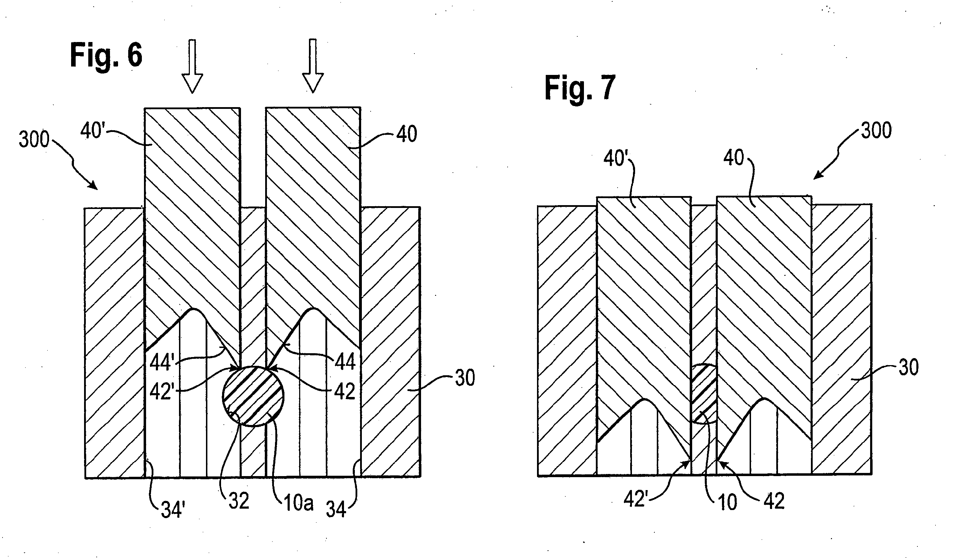

Upon exerting a load on the flexible rod, an inevitable deformation or flexible flow of material will occur in the rod. As depicted in the cross sections of Figs. 6 and 7, a simultaneous downward movement of the punching presses helps to develop a symmetric deformation of the flexible material of the rod during the punching operation. Since bores 34, 34' intersect with the bore 32 off-center from its longitudinal axis on opposite sides thereof, recesses such as shown in Figs. 1-3 may be formed in the rod surface. Both punching presses may therefore be mechanically coupled with each other and further with an operating means. Such operating means may for example be a simple toggle joint. However, any other operating means capable of pressing down the punching presses with suitable force may be employed as well. It is clear, that cutting tools including one or more punching presses may be used.

The separating tool may advantageously be placed or installed in the vicinity of surgeon's operating site, i.e., in a hospital. Accordingly, the surgeon or an attending person may in situ decide where and to what extent recesses shall be applied to the rod.

Hence, costs and efforts can be reduced which are necessary to provide a flexible stabilization device suited for the specific needs of a patient.

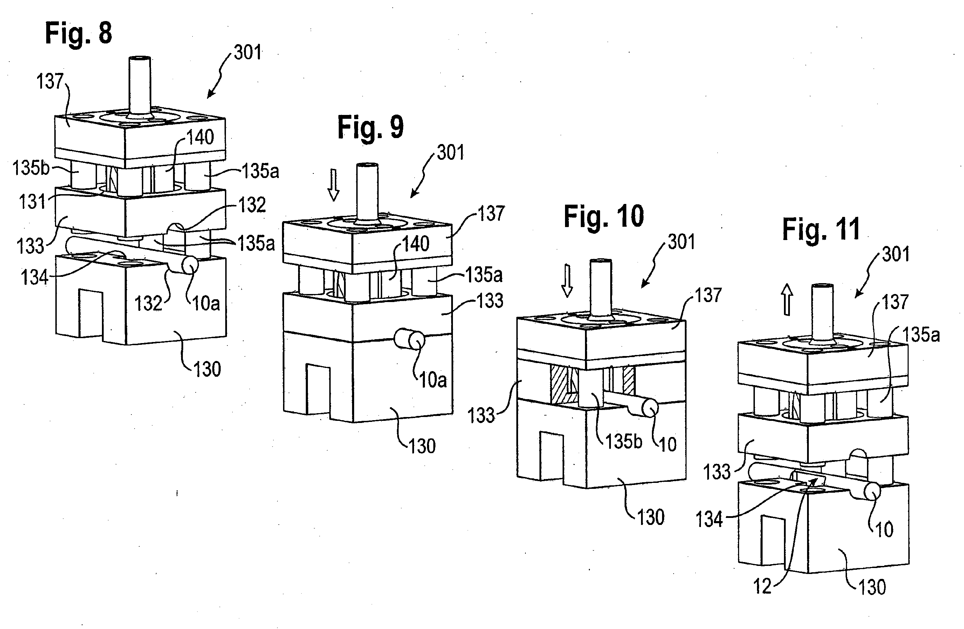

Figs. 8-11 show another embodiment of a tool 301 for removing material from a rod 10a. This embodiment differs from the previous embodiment particularly in that the tool includes a structure which is substantially composed of three plates 130, 133, 137. Other parts not described in detail herein - in particular the shape, orientation and function of the presses - are the same as in the embodiment of Figs. 4-7.

The bottom die plate 130 includes one half of a bore 132 designed to receive the yet un-punched rod 10a. A presser plate 133 is supported by a first spring means (e.g., a coil spring not shown) in a distance above the bottom die plate 130 and includes the other half part of the bore 132 in a bottom face thereof. When the presser plate 133 moves down until the upper face of the bottom die plate 130 is contacted, the rod 10a is held fixed inside the bore 132.

Two guide rods 135a extend upwards from the bottom die plate 130. The presser plate 133 has corresponding bores which receive the guide rods such that the presser plate 133 is held to be movable up and down along the guide rods 135a. The die plate 130 and the presser plate 133 correspond to the socket die of the previous embodiment.

A cutting plate 137 supported in a distance above the presser plate is also guided by the guide rods 135a. The distance between plates 133, 137 is maintained by a second spring mechanism whose spring force is larger than that of the first spring means. Two further guide rods 135b extend downward from the cutting plate 137 in order to be received by corresponding bores of presser plate 133. The presser plate 133 is also movable with respect to the guide rods 135b.

The cutting plate 137 further has - similar to the previous embodiment - two cutting presses extending downward through an opening 131 of the presser plate 133 towards corresponding bores 134 formed in the bottom die plate 130. Bores 132 and 134 intersect each other as in the embodiment shown in Figs. 6-7.

The tools shown in Figs. 4-11 may be fabricated from stainless steel or other suitable materials.

A method of manufacturing a rod 10 as shown in Figs. 1-3 using the tool as described above is now explained with reference to Figs. 8-11:

First, as shown in Fig. 8, the tool is in an uncompressed state, wherein the rod 10a can be inserted into the opened bore 132 on a top face of the bottom die plate 130.

Next, as shown in Fig. 9, the cutting plate 137 is moved down using, e.g. a toggle joint, etc. Since the second spring means requires a larger force to be compressed than the first spring means, the presser plate coincidently moves down along the guide rods 135a until the presser plate contacts the bottom die plate 130 such that the rod 10a is fixed inside the bore 132.

Next, as shown in Fig. 10, the cutting plate is further moved down also against the force of the second spring means. Eventually, the cutting presses 140 along their travel through the bores 134 hit the rod 10a (which is now held fixed by the presser plate) and remove an amount of elastomer material thereof.

Next, as shown in Fig. 11, the load manually exerted on the cutting plate 137 is removed such that the tool 301 returns to the uncompressed state, leaving behind a rod 10 which includes recesses 12. In order to apply further recesses 12 or 12', the rod can then be newly positioned, i.e., shifted and/or rotated.

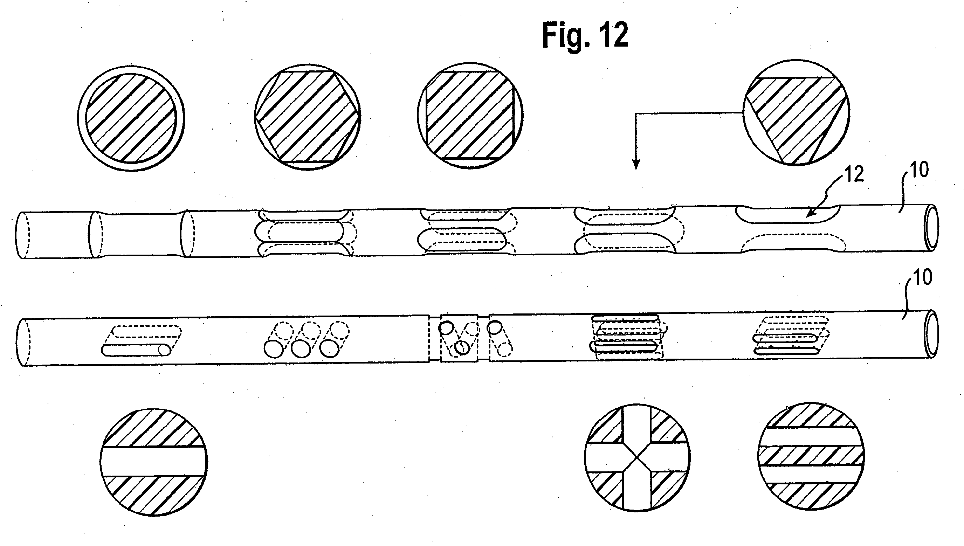

The shapes and sizes of recesses formed in the rod by removing material are not limited to those examples shown in the embodiments above. As shown in Fig. 12, which indicates various kinds of recesses in perspective view along with the associated cross sections of the rod, the recesses may be formed by removing material (a) from the periphery of the rod (upper section of Fig. 12) to yield grooves, or (b) from inner through holes extending through a rod (bottom section of Fig. 12) .

Further, the separation of elastomer material from the rod as proposed herein is not limited to a cutting or punching operation. As shown in the upper left section of Fig. 12, a recess may also be formed by a turning operation in order to yield a rotational symmetric recess. Still further, the directed removal of material as it is achieved in a punching operation may also be accomplished by milling, drilling, water cutting and/or laser cutting. Further, heat may be applied to locally melt and then remove material from the rod.

The rod can have any cross-section in sections other than those comprising the recesses, e.g., cylindrical, hexagonal, square, etc.

According to the invention, recesses are applied to a rod of a flexible stabilization device by removal of material in selected areas. Hence, clamping sections can be maintained between the recesses which may serve to be clamped by bone anchoring devices. The desired bending properties of the rod are thus concentrated in these selective areas outside the clamping sections. As a result, conventional abrasion of elastomer material due to the grinding of a bending rod surface inside a rigid receiving part can be considerably reduced. Consequently, the endurance of the rod can be prolonged.

## Claims

1. A flexible stabilization device for connecting at least two bone anchoring devices (20) which are attachable to vertebrae of the spinal column, comprising:
a flexible rod (10) made of an elastomer material;
the rod having a surface in which at least one first recess (12) is provided which is generated by punching out a material portion of said elastomer material from the rod-shaped element,
**characterized in that** the first recess (12) is provided as a groove comprising a flat portion (16) at a bottom surface thereof.

2. The flexible stabilization device according to claim 1, wherein:
a second recess (12, 12') is formed in the surface;
the second recess is provided as a groove comprising a flat portion (16, 16') at a bottom surface thereof.

3. The flexible stabilization device according to claim 2, wherein the first and second grooves are provided on opposite sides within the same section of the rod.

4. The flexible stabilization device according to claim 2, wherein the first and second groove deviate from to each other with regard to at least one of:
a depth of the flat portion with respect to the surface of the rod in a portion other than the recesses;
a length of the flat portion along a longitudinal axis of the rod; and
an orientation of a surface normal of the flat portion with respect to the longitudinal axis.

5. The flexible stabilization device according to one of claims 3 or 4, wherein the rod comprises:
a clamping section (14) provided for each of the bone anchoring devices, respectively, wherein the rod has a same constant cross section in each of the clamping sections (14);
at least two intermediate section arranged between respectively two of the clamping sections, a first of the intermediate sections comprising the at least one first recess, and a second of the intermediate sections comprising a second recess, which has the at least one differing property from the first recess.

6. The flexible stabilization device according to one of claims 1 to 5, wherein the rod is made from polysiloxane, polyurethane, Poly(styrene-block-isobutylene-block-styrene) (SIBS) or polycarbonate urethane (PCU).

7. A method of manufacturing the rod for connecting at least two bone anchoring devices (20) according to one of claims 1 - 9, comprising:
providing a flexible rod-shaped element (22; 10a) which is made of an elastomer material;
separating a portion of the elastomer material from the flexible rod-shaped element such as to generate at least one first recess (12) in the surface of therein, after the step of providing the rod-shaped element,
wherein the step of separating material includes punching out material from the rod-shaped element, **characterized in that** the first recess (12) is formed as a groove comprising a flat portion (16) at a bottom surface thereof.

## Patentansprüche

1. Eine flexible Stabilisierungsvorrichtung zum Verbinden wenigstens zweier Knochenverankerungsvorrichtungen (20), welche an Wirbeln der Wirbelsäule anbringbar sind, umfassend:
einen flexiblen Stab (10), welcher aus einem Elastomermaterial gebildet ist;
wobei der Stab eine Oberfläche besitzt, in welcher wenigstens eine erste Ausnehmung (12) vorgesehen ist, welche durch Ausstanzen eines Materialabschnitts des Elastomermaterials aus dem stabförmigen Element erzeugt ist,
**dadurch gekennzeichnet, dass** die erste Ausnehmung (12) als eine Einkerbung umfassend einen flachen Abschnitt (16) an deren Bodenoberfläche vorgesehen ist.

2. Die flexible Stabilisierungsvorrichtung gemäß Anspruch 1, wobei:
eine zweite Ausnehmung (12, 12') in der Oberfläche gebildet ist;
wobei die zweite Ausnehmung als eine Einkerbung umfassend einen flachen Abschnitt (16, 16') an deren Bodenoberfläche vorgesehen ist.

3. Die flexible Stabilisierungsvorrichtung gemäß Anspruch 2, wobei die ersten und zweiten Einkerbungen an gegenüberliegenden Seiten innerhalb des gleichen Abschnitts des Stabs vorgesehen sind.

4. Die flexible Stabilisierungsvorrichtung gemäß Anspruch 2, wobei die ersten und zweiten Ausnehmungen voneinander abweichen in Bezug auf wenigstens eines aus:
einer Tiefe des flachen Abschnitts in Bezug auf die Oberfläche des Stabs in einem Abschnitt außerhalb der Ausnehmungen;
einer Länge des flachen Abschnitts entlang einer Längsachse des Stabs; und
einer Orientierung einer Oberflächennormalen des flachen Abschnitts in Bezug auf die Längsachse.

5. Die flexible Stabilisierungsvorrichtung gemäß einem der Ansprüche 3 oder 4, wobei der Stab umfasst:
einen Klemmabschnitt (14), der entsprechend für jede der Knochenverankerungsvorrichtungen vorgesehen ist, wobei der Stab den gleichen konstanten Querschnitt in jedem der Klemmabschnitte (14) aufweist;
wobei wenigstens zwei mittlere Abschnitte zwischen den entsprechend zwei Klemmabschnitten angeordnet sind, wobei ein erster der mittleren Abschnitte wenigstens die erste Ausnehmung umfasst, und ein zweiter der mittleren Abschnitte eine zweite Ausnehmung umfasst, welche wenigstens eine von der ersten Ausnehmung unterschiedliche Eigenschaft aufweist.

6. Die flexible Stabilisierungsvorrichtung gemäß einem der Ansprüche 1 bis 5, wobei der Stab aus Polysiloxan, Polyurethan, Poly(Styrene-Block-Isobutylen-Block-Styrene) (SIBS) oder Polykarbonat-Urethan (PCU) gebildet ist.

7. Ein Verfahren zum Herstellen des Stabs zum Verbinden wenigstens zweier Knochenverankerungsvorrichtungen (20) gemäß einem der Ansprüche 1 bi 9, umfassend:
Bereitstellen eines flexiblen stabförmigen Elements (22; 10a), welches aus einem Elastomermaterial gebildet ist;
Trennen eines Abschnitts des Elastomermaterials von dem flexiblen stabförmigen Element nach dem Schritt des Bereitstellens des stabförmigen Elements, um wenigstens eine erste Ausnehmung (12) in der Oberfläche darin zu erzeugen,
wobei der Schritt des Trennens von Material ein Ausstanzen von Material von dem stabförmigen Element einschließt, **dadurch gekennzeichnet, dass** die erste Ausnehmung (12) als eine Einkerbung ausgebildet ist, die einen flachen Abschnitt (16) an deren Bodenoberfläche umfasst.

## Revendications

1. Dispositif de stabilisation flexible destiné à relier au moins deux dispositifs d'ancrage osseux (20) qui peuvent être fixés à des vertèbres de la colonne vertébrale, comprenant :
une tige flexible (10) réalisée en matériau élastomère ;
la tige ayant une surface dans laquelle au moins un premier évidement (12) est prévu qui est généré par le découpage d'une partie de matériau dudit matériau élastomère à partir de l'élément en forme de tige,
**caractérisé en ce que** le premier évidement (12) est prévu en tant que rainure comprenant une partie plate (16) au niveau d'une surface inférieure de celui-ci.

2. Dispositif de stabilisation flexible selon la revendication 1, dans lequel :
un deuxième évidement (12, 12') est formé dans la surface ;
le deuxième évidement est prévu en tant que rainure comprenant une partie plate (16, 16') au niveau d'une surface inférieure de celui-ci.

3. Dispositif de stabilisation flexible selon la revendication 2, dans lequel les première et deuxième rainures sont prévues sur des côtés opposés dans la même section de la tige.

4. Dispositif de stabilisation flexible selon la revendication 2, dans lequel les première et deuxième rainures s'écartent l'une de l'autre en fonction d'au moins l'une :
d'une profondeur de la partie plate par rapport à la surface de la tige dans une partie autre que les évidements ;
d'une longueur de la partie plate le long d'un axe longitudinal de la tige ; et
d'une orientation d'une normale à la surface de la partie plate par rapport à l'axe longitudinal.

5. Dispositif de stabilisation flexible selon l'une des revendications 3 ou 4, dans lequel la tige comprend :
une section de serrage (14) prévue pour chacun des dispositifs d'ancrage osseux, respectivement, où la tige a une même section transversale constante dans chacune des sections de serrage (14) ;
au moins deux section intermédiaires agencées entre respectivement deux parmi les sections de serrage, une première section des sections intermédiaires comprenant l'au moins un premier évidement, et une deuxième section des sections intermédiaires comprenant un deuxième évidement, qui présente l'au moins une propriété différente du première évidement.

6. Dispositif de stabilisation flexible selon l'une des revendications 1 à 5, dans lequel la tige est réalisée en polysiloxane, polyuréthane, poly(styrène-isobutylènestyrène) (SIBS) ou en polycarbonate uréthane (PCU).

7. Procédé de fabrication de la tige destinée à relier au moins deux dispositifs d'ancrage osseux (20) selon l'une des revendications 1 à 9, comprenant le fait :
de fournir un élément en forme de tige flexible (22 ; 10a) qui est réalisé en matériau élastomère ;
de séparer une partie du matériau élastomère à partir de l'élément en forme de tige flexible de manière à générer au moins un premier évidement (12) dans la surface de celui-ci, après l'étape de fourniture de l'élément en forme de tige,
dans lequel l'étape de séparation de matériau comporte le découpage d'un matériau à partir de l'élément en forme de tige, **caractérisé en ce que** le premier évidement (12) est formé en tant que rainure comprenant une partie plate (16) au niveau d'une surface inférieure de celui-ci.
